# EUROPÄISCHE PATENTSCHRIFT

(11) **EP 1 277 010 B1**
(45) Veröffentlichungstag und Bekanntmachung des Hinweises auf die Patenterteilung: **14.09.2005**
(21) Anmeldenummer: 01914945.9
(22) Anmeldetag: 03.04.2001
(51) Int. Cl.: F16P 1/06, A61F 9/06

(54) **BLENDSCHUTZVORRICHTUNG**
ANTI-GLARE PROTECTION DEVICE
DISPOSITIF DE PROTECTION CONTRE L'EBLOUISSEMENT

(30) Priorität: 22.04.2000 CH 794002000
(43) Veröffentlichungstag der Anmeldung: 22.01.2003
(73) Patentinhaber: OPTREL AG, 9630 Wattwil (CH)
(72) Erfinder: SCHINDELE, Ronald, CH-9200 Gossau (CH)
(74) Vertreter: Frei, Alexandra Sarah
(86) Internationale Anmeldenummer: PCT/CH2001/000213
(87) Internationale Veröffentlichungsnummer: WO 2001/081819

(56) Entgegenhaltungen:
- US-A- 2 152 383
- US-A- 5 042 821
- US-A- 5 377 032
- US-A- 5 959 705

## Beschreibung

Die Erfindung betrifft eine Blendschutzvorrichtung nach dem Oberbegriff des ersten, unabhängigen Patentanspruchs. Die Blendschutzvorrichtung ist insbesondere eine Schweissbrille, eine Schweissmaske oder ein Schweisshelm und weist einen tragenden Teil mit einer Sichtöffnung, eine die Sichtöffnung abdeckende Schutzscheibe und ein hinter der Schutzscheibe angeordnetes, optisches Filterelement auf.

Der tragende Teil von Schweissbrillen, Schweissmasken oder Schweisshelmen dient neben seiner tragenden Funktion dem Schutz des Schweissers gegen mechanische Einwirkungen und gegen Wärmestrahlung. Dieser tragende Teil besteht üblicherweise aus einem Kunststoff, beispielsweise aus Polycarbonat und wird durch Spritzguss oder durch Pressen hergestellt.

Das Filterelement ist in der Sichtöffnung des tragenden Teils angeordnet und dient dem optischen Schutz des Trägers, es stellt also den eigentlichen Blendschutz der Vorrichtung dar. Das Filterelement kann passiv sein, das heisst, es kann beispielsweise aus Schwarzglas bestehen. Das Filterelement kann auch aktiv sein, das heisst, es sperrt oder reduziert den Lichtdurchgang, wenn die Aussenlichtintensität eine vorgegebene Schwelle überschreitet. Als Filterelement dient beispielsweise ein optoelektronisches Element in Form von mindestens einer Flüssigkristallzelle (Liquid-Cristal-Zelle oder LC-Zelle). Zur Detektion der Aussenlichtintensität ist ebenfalls in der Sichtöffnung ein optischer Sensor montiert, dessen Messsignal mit geeigneten Mitteln zu Steuersignalen verarbeitet wird, mit welchen Steuersignalen die optische Durchlässigkeit des Filterelementes gesteuert wird.

Die Schutzscheibe deckt die Sichtöffnung des tragenden Teils, derart, dass das Filterelement und gegebenenfalls der optische Sensor hinter der Schutzscheibe, das heisst, in getragenem Zustand zwischen der Schutzscheibe und den Augen des Schweissers positioniert sind. Die Schutzscheibe ist transparent und dient dem Schutz des optischen Filterelementes vor Verschmutzung und Beschädigung. Die Schutzscheibe besteht vorteilhafterweise aus Kunststoff, beispielsweise aus Polycarbonat und ist fest oder auswechselbar am tragenden Teil montiert. Üblicherweise ist für diese Montage der tragende Teil und/oder die Schutzscheibe mit Verbindungsmitteln ausgerüstet, derart, dass die Schutzscheibe beispielsweise durch Einschnappen montiert werden kann und dass sie in montiertem Zustand mehr oder weniger gegen den tragenden Teil gepresst oder mit diesem verspannt ist.

Es zeigt sich, dass Filterelemente und für aktive Filterelemente notwendige Sensoren derartiger Blendschutzvorrichtungen, auch wenn sie durch die Schutzscheibe gegen aussen geschützt sind, relativ schnell verschmutzen und dadurch ihre Funktionssicherheit verlieren. Es zeigt sich auch, dass ein zusätzlicher Schutz des Trägers gegen beim Schweissen entstehende Gase und Rauch wünschenswert wäre.

In US 5,377,032 ist eine Schweisserschutzmaske beschrieben, bei welcher in einer Sichtöffnung eines tragenden Teils eine Filtereinheit zwischen Schutzscheiben angeordnet ist. Eine äussere Schutzscheibe deckt auch einen optischen Sensor zur Steuerung der Durchlässigkeit der Filtereinheit ab, sowie einen Schalter zum Ein/Ausschalten der Filtereinheit. Die Schutzscheibe liegt an den äusseren Rändern teilweise auf der Filtereinheit auf, ist aber in einem Randbereich der den Sensor und den Schalter enthält, von der Filtereinheit beabstandet. So kann der Schalter durch Druck auf die Schutzscheibe betätigt werden.

Die US 2,152,383 beschreibt eine Schweisserschutzmaske, bei welcher zur Verhinderung von Streulicht eine Dichtung um den Rand einer Schutzscheibe und einer passiven Filterscheibe verläuft. Zur vollständigen Abschirmung von Licht verläuft die Dichtung sowohl zwischen einem Gehäuse und der Schutzscheibe als auch zwischen der Schutzscheibe und der Filterscheibe. Dadurch wird verhindert, dass Licht, welches durch eine Stirnfläche der Schutzscheibe seitlich abgeleitet wird, die Filterscheibe an deren Rand umgeht. Dazu wird die Dichtung aus einem speziell geformten Gummiprofil hergestellt.

Die Erfindung stellt sich deshalb die Aufgabe, eine Blendschutzvorrichtung mit einem tragenden Teil, mit einer Schutzscheibe und mit einem hinter der Schutzscheibe angeordneten, optischen Filterelement zu schaffen, welche Blendschutzvorrichtung mit einem minimalen herstellungstechnischen Mehraufwand einen besseren Schutz des Filterelementes und im Falle eines aktiven Filterelements des dazugehörenden Sensors vor Verschmutzung und gleichzeitig einen erhöhten Schutz des Trägers vor Gasen, Rauch und Schmutz bietet, als dies mit entsprechenden Vorrichtungen gemäss dem Stande der Technik möglich ist.

Diese Aufgabe wird gelöst durch die Blendschutzvorrichtung, wie sie durch die Ansprüche definiert wird.

Die erfindungsgemässe Blendschutzvorrichtung weist zwischen tragendem Teil und Schutzscheibe oder zwischen Filterelement und Schutzscheibe eine rund um die Sichtöffnung verlaufende Dichtung auf. Es zeigt sich, dass durch diese Dichtung Verschmutzung und unerwünschte Gase im Inneren der Blendschutzvorrichtung zum grössten Teil verhindert werden kann. Die genannte Dichtung stellt also eine sehr einfache aber trotzdem sehr effektive Verbesserung von bekannten derartigen Blendschutzvorrichtungen dar.

Die Dichtung der erfindungsgemässen Blendschutzvorrichtung besteht aus einem elastisch komprimierbaren Material und ist derart zu konzipieren, dass in jedem Zustand der Blendschutzvorrichtung die Schutzscheibe im wesentlichen gasdicht am tragenden Teil oder am Filterelement anliegt. Die Dichtung wirkt auch stossdämpfend und bewirkt, dass die Schutzscheibe mechanische Einwirkungen wie Schläge eher unbeschadet überstehen kann.

Der Unterschied zwischen der erfindungsgemässen Blendschutzvorrichtung und einer entsprechenden Blendschutzvorrichtung gemäss dem Stande der Technik, in der die Schutzscheibe durch Aufschnappen zwar ebenfalls am tragenden Teil anliegen kann, in keiner Weise aber gasdicht anliegt, besteht also in einer elastisch komprimierbaren Dichtung, die zwischen Schutzscheibe und tragendem Teil oder zwischen Schutzscheibe und Filterelement rund um die Sichtöffnung angeordnet ist und die derart konzipiert ist, dass sie Gaseintritt hinter die Schutzscheibe nicht nur im neuen und unbelasteten Zustand der Blendschutzvorrichtung sondern auch in gebrauchtem, das heisst deformierendem Verschleiss ausgesetztem Zustand und während dem Tragen, wenn der tragende Teil gegebenenfalls durch mechanische Spannungen oder Wärmespannungen gegenüber der Schutzscheibe verschoben oder verspannt ist, im wesentlichen zu unterbinden vermag.

Vorteilhafterweise ist die Dichtung am tragenden Teil, am Filterelement oder an der Schutzscheibe befestigt und besteht aus einem Kunststoff, vorteilhafterweise aus einem geschäumten Kunststoff, beispielsweise aus geschäumtem Polyurethan oder aus einem geeigneten Silikonkunststoff.

Die Erfindung wird anhand der folgenden Figuren im Detail beschrieben. Dabei zeigen:
- **Figuren 1 und 2**: eine auswechselbare Schutzscheibe für eine Schweissbrille, für eine Schweissmaske oder für einen Schweisshelm, auf welcher Schutzscheibe eine Dichtung aufgebracht ist (Figur 1: Frontansicht; Figur 2: Schnitt A-A);
- **Figuren 3 bis 5**: drei beispielhafte Ausführungsformen von Dichtungen in der erfindungsgemässen Blendschutzvorrichtung.

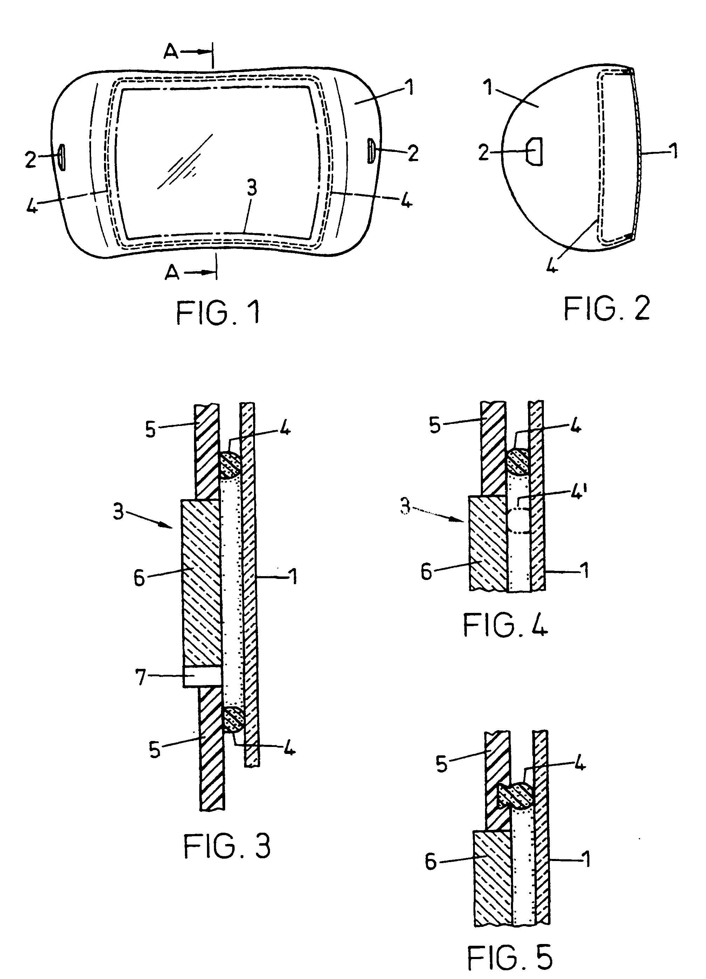

**Figuren 1 und 2** zeigen eine beispielhafte Schutzscheibe 1 für eine erfindungsgemässe Blendschutzvorrichtung, die Figur 1 als Frontansicht, die Figur 2 als Schnitt A-A. Die Schutzscheibe ist seitlich nach hinten gewölbt und weist seitliche Schlitze 2 auf, mit deren Hilfe die Schutzscheibe an einem tragenden Teil, der nicht dargestellt ist, befestigt, beispielsweise aufgeschnappt wird. Vom tragenden Teil ist in der Figur 1 als strichpunktierte Linie der Umriss der Sichtöffnung 3 dargestellt. Rund um diese Sichtöffnung 3 verläuft eine an der Schutzscheibe befestigte Dichtung 4.

**Figur 3** zeigt als Detail einen Schnitt durch Schutzscheibe 1, tragenden Teil 5 mit Sichtöffnung 3 und durch ein in der Sichtöffnung 3 angeordnetes Filterelement 6 mit Sensor 7. Zwischen Schutzscheibe 1 und tragendem Teil 5 ist die Dichtung 4 sichtbar, die im dargestellten Falle am tragenden Teil 5 befestigt ist.

**Figur 4** zeigt einen ähnlichen Schnitt wie Figur 3, wobei die dargestellte Dichtung 4 in diesem Falle an der Schutzscheibe 1 befestigt ist. Strichpunktiert ist die Dichtung 4' auch in einer Position dargestellt, in der sie nicht zwischen tragendem Teil 5 und Schutzscheibe 1 sondern zwischen dem optischen Filterelement 6 und der Schutzscheibe 1 angeordnet ist. Auch in diesem Falle verläuft die Dichtung 4' im wesentlichen rund um die Sichtöffnung 3 bzw. im wesentlichen parallel zum Rand der Sichtöffnung 3 des tragenden Teils 5.

Vorteilhafterweise wird eine Dichtung 4, wie sie in den Figuren 3 und 4 dargestellt ist, dadurch hergestellt, dass das Kunststoffmaterial für die Dichtung direkt auf den tragenden Teil 5 bzw. auf die Schutzscheibe 1 oder das Filterelement 6 extrudiert und dabei geschäumt wird. In dieser Weise entsteht zwischen dem Kunststoff des tragenden Teiles 5, der Schutzscheibe 1 oder des Filterelementes 6 eine stoffschlüssige Verbindung, die einer Verschweissung oder Verklebung ähnlich ist und durch die die Dichtung 4 vollauf genügend mit dem tragenden Teil 5 bzw. mit der Schutzscheibe 1 oder dem Filterelement 6 verbunden ist.

**Figur 5** zeigt noch eine weitere Ausführungsform einer Dichtung 4, die formschlüssig in einer entsprechenden Nut am tragenden Teil 5 befestigt ist. Diese Dichtung ist gegebenenfalls ein im Handel erhältlicher O-Ring. Auch eine derartige Dichtung kann anstatt am tragenden Teil 5 an der Schutzscheibe 1 oder am Filterelement 6 angeordnet sein.

## Patentansprüche

1. Blendsrhutzvorrichtung mit einem tragenden Teil (5), mit einem aktiven, optischen Filterelement (6), das in einer Sichtöffnung (3) des tragenden Teils (5) angeordnet ist und dessen Durchlässigkeit von einem optischen Sensor (7) gesteuert wird, und mit einer Schutzscheibe (1), die, das optische Filterelement (6) und den Sensor (7) deckend, am tragenden Teil (5) befestigt ist oder befestigbar ist, **dadurch gekennzeichnet, dass** zum Schutz von Filterelement (6) und Sensor (7) vor Gasen, Rauch und Schmutz zwischen tragendem Teil (5) und Schutzscheibe (1) oder zwischen Filterelement (6) und Schutzscheibe (1) eine sich im wesentlichen rand um die Sichtöffnung (3) und den Sensor (7) erstreckende Dichtung (4, 4') angeordnet ist, die aus einem elastisch komprimierbaren Material besteht, die am tragenden Teil (5), an der Schutzscheibe (1) oder am optischen Filterelement (6) befestigt ist und durch die ein Gasdurchtritt zwischen dem tragenden Teil (5) und der Schutzscheibe (1) oder zwischen dem Filterelement (6) und der Schutzseheibe (1) mindestens erschwert wird.

2. Blendschutzvorrichtung nach Anspruch 1, **dadurch gekennzeichnet, dass** die Dichtung (4) stoffschlüssig befestigt ist.

3. Blendschutzvorrichtung nach Anspruch 2, **dadurch gekennzeichnet, dass** die Dichtung (4) auf den tragenden Teil oder das Filterelement oder die Schutzscheibe extrudiert und **dadurch** mit diesem/dieser verklebt ist.

4. Blendschutzvorrichtung nach einem der Ansprüche 1 bis 3, **dadurch gekennzeichnet, dass** die Dichtung am tragenden Teil (5) oder am optischen Filterelement (6) befestigt ist.

5. Blendschutzvorrichtung nach einem der Ansprüche 1 bis 4, **dadurch gekennzeichnet, dass** die Dichtung aus einem geschäumten Kunststoff besteht.

6. Biendschutzvorrichtung nach einem der Ansprüche 1 bis 5, **dadurch gekennzeichnet, dass** die Dichtung (4) aus Polyurethan oder aus einem Silikonkunststoff besteht.

7. Blendschutzvorrichtung nach einem der Ansprüche 1 bis 6, **dadurch gekennzeichnet, dass** das Filterelement (6) mindestens ein Flüssigkristallelement aufweist und Mittel zur Ansteuerung des mindestens einen Flüssigkristallelementes unter Verwendung der Signale des Sensors (7).

## Claims

1. Anti-glare protection device comprising a supporting part (5), an active optical filter element (6) arranged within a sight opening (3) of the supporting part (5) and with a transmittance being controlled by an optical sensor (7), and a protective plate (1) affixed to or affixable to the supporting part (5) such as to cover the optical filter element (6) and the sensor (7), **characterised in that** for protecting the filter element (6) and the sensor (7) against gases, smoke and dirt, a seal (4, 4') substantially extending around the sight opening (3) and the sensor (7) is arranged between the supporting part (5) and the protective plate (1) or between the filter element (6) and the protective plate (1), the seal consisting of an elastically compressible material and being affixed to the supporting part (5), to the protective plate (1) or to the optical filter element (6) and rendering at least difficult the passage of gas between the supporting part (5) and the protective plate (1) or between the filter element (6) and the protective plate (1).

2. Anti-glare protection device in accordance with claim 1, **characterised in that** the seal (4) is affixed with a direct material to material connection.

3. Anti-glare protection device in accordance with claim 2, **characterised in that** the seal (4) is extruded onto, and in this manner glued to, the supporting part or the optical filter element or the protective plate.

4. Anti-glare protection device according to one of claims 1 through 3, **characterised in that** the seal is affixed to the supporting part (5) or the optical filter element (6).

5. Anti-glare protection device according to one of claims 1 through 4, **characterised in that** the seal consists of a foamed plastic material.

6. Anti-glare protection device in accordance with one of claims 1 through 5, **characterised in that** the seal (4) consists of polyurethane or of a silicone plastic material.

7. Anti-glare protection device according to one of claims 1 through 6, **characterised in that** the filter element (6) comprises at least one liquid crystal element and means for controlling the at least one liquid crystal element by making use of the signal of the optical sensor (7).

## Revendications

1. Dispositif anti-éblouissement avec un élément porteur (5), avec un élément filtrant optique actif (6) qui est disposé dans une échancrure (3) de l'élément porteur (5) et dont la transparence est contrôlée par un capteur optique (7) et avec un écran protecteur (1) fixé ou pouvant être fixé sur l'élément porteur (5), tout en couvrant l'élément filtrant optique (6) et le capteur (7), **caractérisé en ce que** pour protéger l'élément filtrant (6) et le capteur (7) contre les gaz, la fumée et les saletés, un joint (4, 4') en un matériau élastiquement comprimable, s'étendant sensiblement tout autour de l'échancrure (3) et du capteur (7), qui est fixé sur l'élément porteur (5), sur l'écran protecteur (1) ou sur l'élément filtrant (6) et qui rend au moins plus difficile la pénétration de gaz entre l'élément porteur (5) et l'écran protecteur (1) ou entre l'élément filtrant (6) et l'écran protecteur (1) est disposé entre l'élément porteur (5) et l'écran protecteur (1) ou entre l'élément filtrant (6) et l'écran protecteur (1).

2. Dispositif anti-éblouissement selon la revendication 1, **caractérisé en ce que** le joint (4) est fixé par liaison de matière.

3. Dispositif anti-éblouissement selon la revendication 2, **caractérisé en ce que** le joint (4) est extrudé sur l'élément porteur ou sur l'élément filtrant ou sur l'écran protecteur et de ce fait, il est collé sur ce/ces dernier(s).

4. Dispositif anti-éblouissement selon l'une quelconque des revendications 1 à 3, **caractérisé en ce que** le joint est fixé sur l'élément porteur (5) ou sur l'élément filtrant optique (6).

5. Dispositif anti-éblouissement selon l'une quelconque des revendications 1 à 4, **caractérisé en ce que** le joint est en une matière plastique expansée.

6. Dispositif anti-éblouissement selon l'une quelconque des revendications 1 à 5, **caractérisé en ce que** le joint (4) est en polyuréthane ou en une matière plastique silicone.

7. Dispositif anti-éblouissement selon l'une quelconque des revendications 1 à 6, **caractérisé en ce que** l'élément filtrant (6) comporte au moins un élément à cristaux liquides et des moyens d'activation d'au moins l'élément à cristaux liquides, par utilisation des signaux du capteur (7).
